# EUROPEAN PATENT SPECIFICATION

(11) **EP 1 527 086 B1**
(45) Date of publication and mention of the grant of the patent: **09.01.2008**
(21) Application number: 03792179.8
(22) Date of filing: 04.07.2003
(51) Int. Cl.: C07K 7/06, C07K 14/705, C12N 15/67, A61K 48/00

(54) **COMPOUNDS BINDING TO P-SELECTIN**
VERBINDUNGEN DIE P-SELECTIN BINDEN
COMPOSES SE LIANT A LA P-SELECTINE

(30) Priority: 09.08.2002 EP 02078308
(43) Date of publication of application: 04.05.2005
(73) Proprietor: Astellas Pharma Europe B.V., 2353 EW Leiderdorp (NL)
(72) Inventor: APPELDOORN, Chantal, Catharina, Maria LACDR, NL 2300 RA Leiden (NL); BIESSEN, Erik, Anna, Leonardus, NL 2300 RA Leiden (NL); MOLENAAR, Thomas, Jacobus, Maria, NL 2300 RA Leiden (NL); KUIPER, Johan LACDR, P.O.Box 9502 NL-2300 RA Leiden (NL); VAN BERKEL, Theodorus, Josephus, Cornelis, P.O.Box 9502 NL 2300 RA Leiden (NL)
(86) International application number: PCT/EP2003/007260
(87) International publication number: WO 2004/018502

(56) References cited:
- WO-A-00/27420
- US-A- 5 916 876
- MOLENAAR TOM J M ET AL: "Specific inhibition of P-selectin-mediated cell adhesion by phage display-derived peptide antagonists." BLOOD. UNITED STATES 15 NOV 2002, vol. 100, no. 10, 15 November 2002 (2002-11-15), pages 3570-3577, XP002223456 ISSN: 0006-4971

## Description

The present invention relates to compounds which bind selectively to the adhesion molecule human P-selectin, to methods for preparing such compounds, to the use of such compounds in therapeutic or diagnostic methods and in pharmaceutical compositions, to binding molecules binding to said compounds, and to a method for determining whether a compound is capable of binding to P-selectin.

### BACKGROUND OF THE INVENTION

In recent years, cell surface adhesion molecules have become recognised as key mediators in numerous cellular processes including cell growth, differentiation, immune cell transmigration and response and cancer metastasis.

Four major categories of adhesion molecules have been identified: the immunoglobulin superfamily cell adhesion molecules (CAMs), cadherins, integrins and selectins.

The selectins represent a family of presently three transmembraneous, carbohydrate-binding glycoproteins: "endothelial" E-selectin, "leukocyte" L-selectin and "platelet" P-selectin. All three selectins are divalent cation (e.g. calcium) dependent and possess an extracellular domain with a carbohydrate recognition motif, an epidermal growth factor-like motif, and some smaller domains related to complement-regulatory proteins.

Human P-selectin (also referred to as GMP-140, LECAM-3, PADGEM, CD62 and . CD62P) is expressed by platelets and endothelial cells. When expressed on the surfaces of these cells, its most notable effect is the slowing of leukocytes as these leave the capillaries and enter the postcapillary venules, the latter representing the major site of leukocyte-endothelium adhesion. The slowing process is observed as leukocyte rolling, signifying an initial adhesion with relatively low affinity. The firm adhesion of rolling leukocytes is primarily mediated by integrins.

In endothelial cells, P-selectin is stored in Weibel-Palade bodies; in platelets, it is found in the α-granules. Following activation, P-selectin is mobilised to the cell surfaces within a few minutes in response to a variety of inflammatory or thrombogenic agents. The endothelial P-selectin's primarily function is to recruit leukocytes into postcapillary venules, while platelet P-selectin also results in the formation of thrombi. One of the presently known natural ligands of P-selectin is PSGL-1 (P-selectin glycoprotein ligand-1), a 120 kDa sialoprotein expressed on the surface of leukocytes where it is concentrated at the uropod. More detailed descriptions of the structure and functions of P-selectin are found in numerous publications (e.g. J. Panes, Pathophysiology 5: 271 (1999); F. Chamoun et al., Frontiers in Bioscience 5: e103 (Nov. 1, 2000) and S.-I. Hayachi, Circulation 102: 1710 (2000)).

Inflammation and inflammatory processes play a major role in the pathophysiology of numerous diseases and conditions. Conditions of the brain in which increased selectin levels were found and which may therefore involve selectin-mediated pathophysiological events, include severe traumatic brain injury, relapsing-remitting multiple sclerosis, cerebral artery occlusion, ischemia and stroke. Conditions of the heart in which selectins are suggested to play a role include acute myocardial infarct, arterial injury, such as produced by angioplasty, and ischemia. Similarly, selectins are involved in conditions of the kidneys, such as renal injury from ischemia and reperfusion, and renal failure. Furthermore, selectins appear to play a role in organ transplant rejection, cold ischemia, hemorrhagic shock, septic shock, tumour metastasis, chronic inflammation, rheumatoid arthritis, inflammatory bowel disease, atherosclerosis, restenosis, angiogenesis, disseminated intravascular coagulation, adult respiratory distress syndrome and circulatory shock.

Thus, it would seem feasible to improve these and other conditions involving the activation of endothelial cells and leukocytes and specifically the mobilisation and expression of P-selectin by specifically interrupting the P-selectin cascades. This can be done, for instance, by the administration of ligands which selectively bind to human P-selectin, but which do not possess its bioactivity. By this method, mobilised P-selectin could be inactivated and leukocyte-induced tissue damage prevented. Potentially, the same effect could be achieved by gene therapy, provided the P-selectin ligand or antagonist is a peptide or modified peptide. According to this method, somatic cells of a person in need of the therapy would be transfected with an expression vector carrying a DNA sequence encoding a P-selectin antagonist.

P-selectin ligands or antagonists may also be used for the prevention of diseases and conditions described above. Furthermore, such ligands may also be useful in the *in vivo* or *in vitro* diagnosis of these diseases.

Various attempts have been made in recent years to identify or create such selective ligands to P-selectin. So far, a number of substances were tested, but no clinical studies have yet provided conclusive evidence that any of these compounds produce the desired clinical effects while being tolerable in terms of side effects.

For instance, antibodies to P-selectin that were produced and tested in animal models, were found to protect kidneys from ischemic-reperfusion injury (H.C. Rabb et al., JASN 5 : 907 (1997) and US-A-6,033,667). In another study, a recombinant soluble form of P-selectin glycoprotein ligand-1 (rPSGL-Ig) was used to inhibit thrombosis in cats (M.J. Eppihimer et al., Arteriosclerosis, Thrombosis, and Vascular Biology 20: 2483 (2000)). WO-96/09309 discloses oligosaccharide structures that are ligands to E- and P-selectin. WO-99/41363 discloses podocalyxin-like proteins that bind to selectins. WO-00/41711 describes various smaller peptides or peptide sequences that bind to members of the human selectin family; most of the sequences comprise one or more units of leucine or isoleucine.

As another approach to inhibit the P-selectin cascade, various peptides derived from the lectin domain of the selectin family were found to inhibit neutrophil adhesion to P-selectin (e. g. US-A-6,111,065 and US-A-5,916,876); these peptides probably bind to P-selectin receptors on leukocytes.

In WO-94/05269, peptides are described which inhibit binding of selectins such as P-selectin, E-selectin and L-selectin. These peptides have as their core region portions of the 11-18 amino acid sequence of P-selectin, E-selectin or L-selectin. Further, WO-95/31210 relates to peptides and compounds that bind selectins including endothelium leukocyte adhesion molecule 1 (ELAM-1). These peptides are used for blocking adhesion of leukocytes to the selectins, i.e. especially E-selectin, but also P-selectin or L-selectin, for the purpose of inhibiting inflammation.

In not pre-published European patent application No. 01203314.8 compounds, which comprise the peptidic sequence XAₓA₃A₁A₂A₁Y or a functional equivalent thereof and have selective affinity to human P-selectin, have been disclosed wherein:
A₁ is a D- or L-cysteine (C), D- or L-methionine (M), D- or L-valine (V) or an analogue thereof;
A₂ is a D- or L- aspartic acid (D) or an analogue thereof;
A₃ is a D- or L- phenylalanine (F), D- or L-tyrosine (Y) or D- or L-tryptophan (W) or an analogue thereof;
Aₓ is a D- or L-amino acid;
X marks the N-terminal side of said sequence and
Y marks the C-terminal side of said sequence or
X and Y together can form a cyclic system.

The compound EWVDV described in the above-mentioned application has an affinity for P-selectin, as expressed by an IC₅₀-value of about 2 µM. Its tetramer on streptavidin has an IC₅₀ of about 2 µM.

A similar disclosure is given in Molenaar et al, 2002, Blood 100, pp.3570-3577.

Despite the above-mentioned efforts, there is still a need for substances with selective affinity to P-selectin, which can be used for preparing pharmaceutical compositions for the diagnosis, prevention and treatment of various diseases and conditions involving the adherence of leukocytes to vascular endothelial cells or to platelets. For *in vivo* use it would be highly desirable to have relatively simple compounds with a very high affinity for P-selectin. There is also a need for P-selectin ligands, which can be used as targeting molecules or moieties in pharmaceutical compositions for the targeting of drugs or genetic material to tissues expressing P-selectin.

### SUMMARY OF THE INVENTION

It is therefore an object of the invention to provide compounds with selective affinity to human P-selectin.

In particular, it is an object of the invention to provide compounds which act as antagonists or partial antagonists of P-selectin.

It is another object of the invention to provide compounds which act as targeting ligands with an ability to target drugs and genetic material to cells and tissues expressing P-selectin.

A further object of the invention is the provision of methods for preparing such compounds.

Yet another object is the presentation of uses of such compounds, and of compositions which contain the compounds.

### LEGENDS TO THE FIGURES

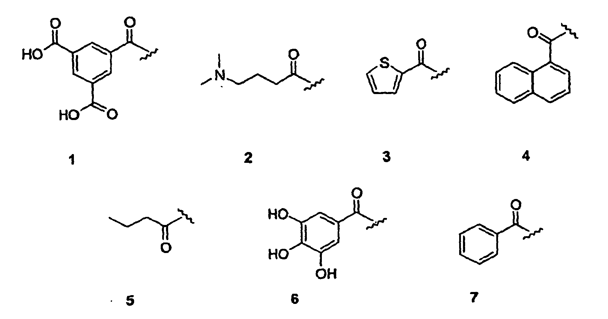

Figure 1 shows the chemical structures of acyl moieties 1-7 introduced to core sequence compound 8.

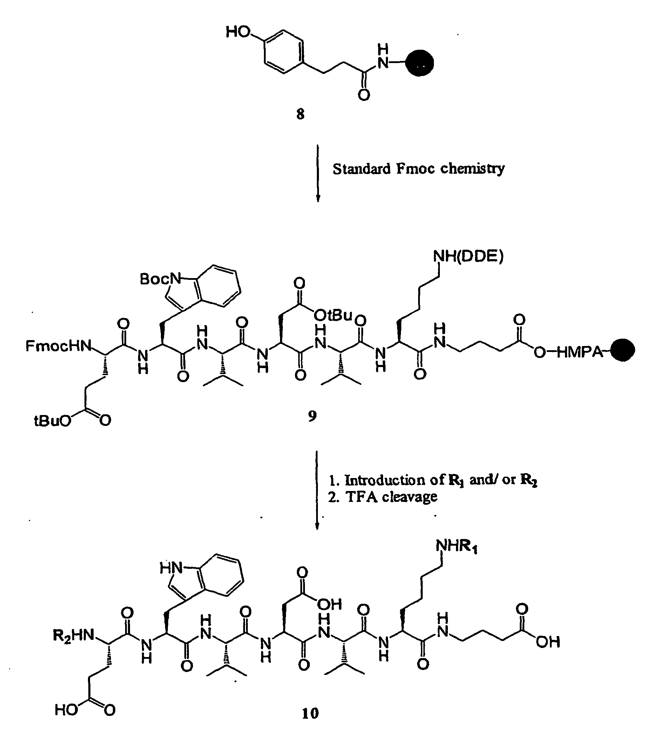

Figure 2 is a schematic representation of the solid phase synthesis of compound library 10. Core sequence compound S was synthesised using standard Fmoc chemistry on Tentagel S-NH₂ resin with HMPA linker. The core peptide was then derivatised at the N-terminus (after removal of the Fmoc) or on the C-terminus (after selective removal of the DDE group by 2% hydrazine) with 7 different carboxylic acids (see figure 1). The peptides were liberated from the resin by TFA cleavage resulting in compound library **10**. *Fmoc, N-*(9-fluoroenyl)methoxy-carbonyl; *HMPA,* 4-hydroxymethylphenoxyacetic acid; *Boc,* N-(t-butoxycarbonyl); *tBu*, tert butyl; *TFA,* trifluoro acetic acid; *DDE,* 4,4-dimethyl-2,6-dioxocyclohex-1-ylidene)ethyl.

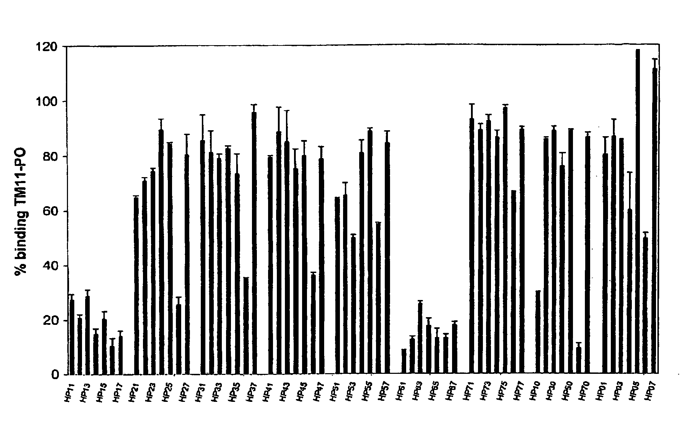

Figure 3 is a graphic representation of the ability of derivatised peptides HP*10-*HP77 to inhibit ligand binding to human P-selectin. Crude derivatised peptides were tested in a competition assay for their ability to displace binding of TM11-PO to human P-selectin. Each bar represents the binding of TM11-PO in the presence of derivatised peptide (5 µM, n=3-5). Peptides are coded HP*ij* at which *i* and *j* refer to the acyl moiety 1-7, as shown in Figure I and attached to the N- and C-terminus, respectively. N/C-Unmodified amino groups are indicated by 0.

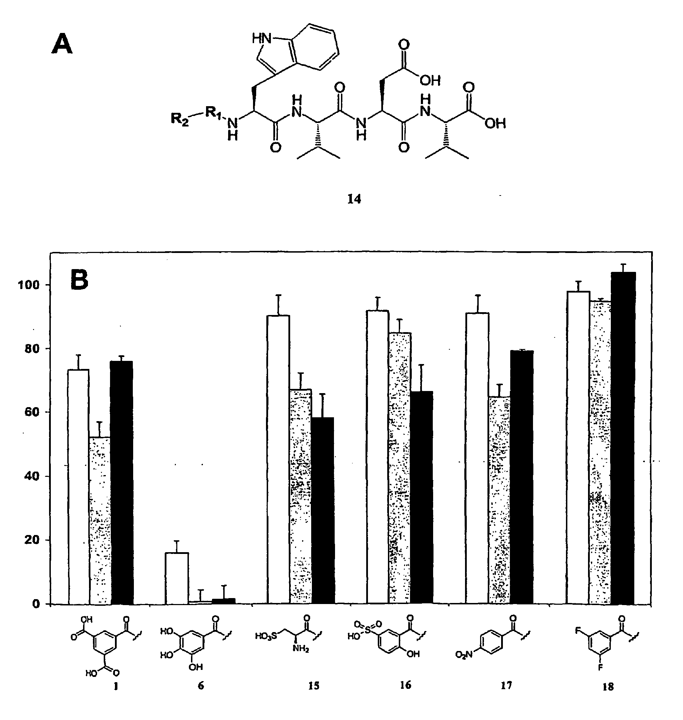

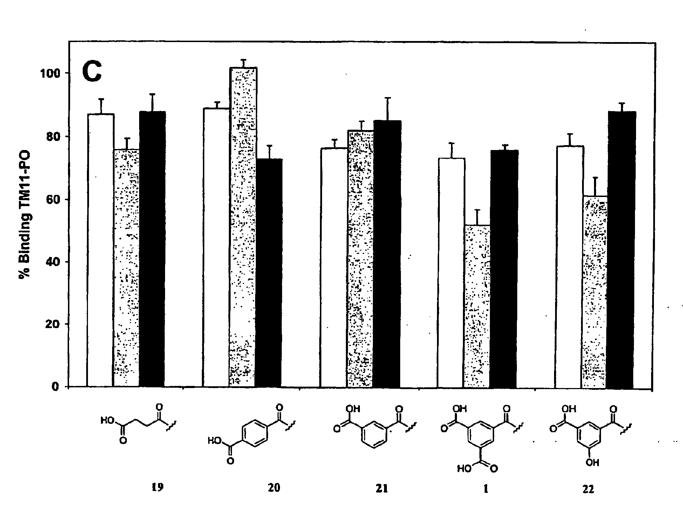

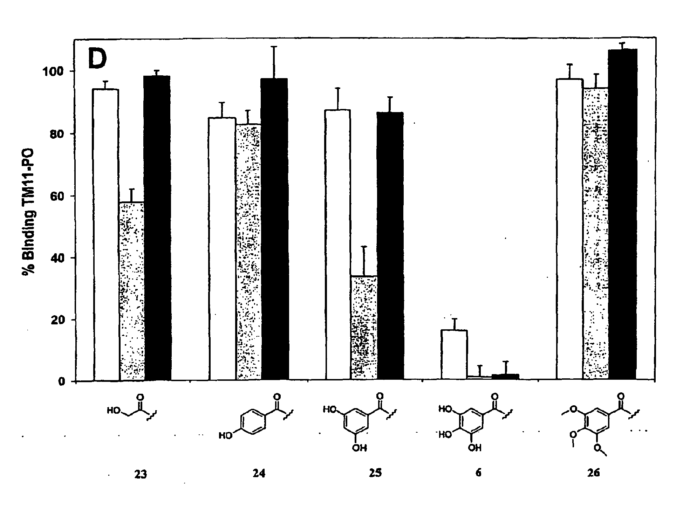

Figures 4-6 show the chemical structure (figure 4A) and biological activity of compound library 14 (figures 4B,5C and 6D). Crude peptide library 14 (1 µM) was tested in a competition assay of TM11-PO binding to human P-selectin. Three different spacers between the acyl moiety and the WVDV core sequence were used (R₁): no spacer (white bars), a glycyl spacer (grey bars) and amino butyric spacer (black bars). The chemical structure of the introduced acyl modification (R₂) is depicted below these bars.

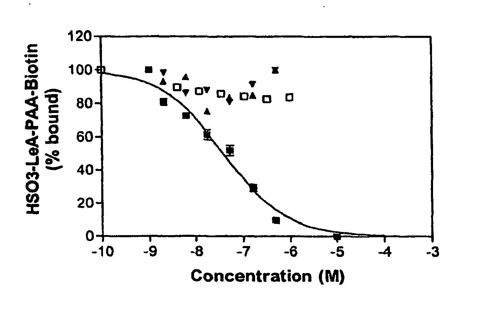

Figure 7 shows a graphical representation of the competition of biotin-PAA-Le^{a}-SO₃ binding to human P-selectin (■), mouse P-selectin (□), human L-selectin (▲) and human E-selectin (▼) by peptide 28. Wells were coated with each selectin (0.3 µg/ ml) and incubated with 0.33 µg/ml biotin-PAA-Le^{a}-SO₃ with or without peptide 28 as described in example 3.

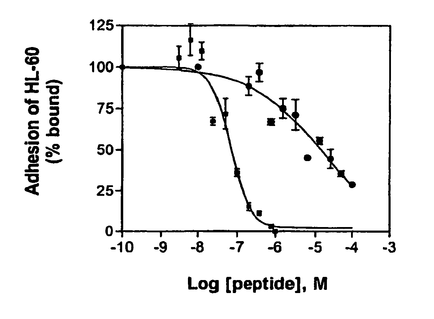

Figure 8 is a graphical representation of the competition of HL-60 cell binding to CHO-P cells by peptide 28 (■) or EWVDV (•). CHO-P cells seeded in 96 wells were incubated with calcein labeled HL-60 cells in the presence of peptide 28 or EWVDV. Data points are means of 12 data points in quadruplicate.

### DETAILED DESCRIPTION OF THE INVENTION

The compounds of the present invention have selective affinity to human P-selectin and are derivatives of peptides represented by X(Aₓ)ₘA₃A₁A₂A₁Y, wherein:
- A₁ is a D- or L- cysteine (C), or a D- or L- valine (V) ;
- A₂ is a D- or L- aspartic acid (D);
- A₃ is a D- or L- phenylalanine (F), or a D-
- tryptophan (W);
- Aₓ is a D- or L-amino acid selected from the group consisting of glutamic acid (E) aspartic acid (D), glycine (G) and cysteine (C);
- X marks the N-terminal side of said sequence and is hydrogen or a residue comprising 1 to 6 D- or L-amino acids or analogues thereof;
- Y marks the C-terminal side of said sequence and is-OH or a reside comprising 1 to 11 D-or L-amino acids or analogues thereof; wherein X and Y together may form a cyclic system; characterised in that either X or Y, or both X and Y, is substituted with the group:
- R¹-(Z)ₙ- wherein:
- Z is -CO-, and wherein
- R¹ is selected from:
- a (C₂-C₈)alkyl group in which at least one C-atom is replaced with a nitrogen atom;
- a (C₆-C₁₄)aryl group which may be substituted with at least one group selected from -OH or -COOH;
and wherein m and n represent integers independently selected from 0 and 1.

Peptides are defined as amides that are derived from two or more amino acids by combination of the amino group of one acid with the carboxyl group of another (Merriam Webster Medical Dictionary ^{©}2001). As used herein, a peptide may also refer to a peptidic structure within a molecule. Typically, peptides are composed of naturally occurring L-α-amino acids, which are alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysine (Lys or K), methionine (Met or M), phenylalanine (Phe or F), proline (Pro or P), serine (Ser or S), threonine (Thr or T), tryptophan (Trp or W), tyrosine (Tyr or Y), and valine (Val or V).

According to the invention, the two units of A₁ within the sequence are selected independently, they can be identical or different from each other. Preferably, at least one of the A₁ units represents valine (V). More preferably, both A₁ units are valine (V). In another embodiment, A₁ is D- or L *-* cysteine(C) or valine (V).

Particularly, the invention also encompasses a method for determining whether a compound is capable of binding to human P-selectin, comprising substituting in a compound according to the invention, an amino acid for a conservative amino acid and determining whether the resulting compound is capable of binding to said P-selectin. According to the invention, A₂ is a D- or L-aspartic acid with a solvent exposed side chain bearing a negative charge at physiological pH.

A₃ is a D- or L-amino acid with an aromatic side chain selected from the group consisting of phenylalanine (F), and tryptophan (W). More preferably, A₃ is an L-amino acid selected from this group. Most preferred is tryptophan (W).

Aₓ is a D- or L-amino acid, as defined above, selected from the group consisting of glutamine acid (E), aspartic acid (D), glycine (G) and cysteine (C). In one of the preferred embodiments, Aₓ is a D- or L- glutamic acid (E).

It has to be noted that the 2 units of A₁ are selected independently, *i*.*e*. they can be identical or different from each other.

X marks the N-terminal group. X can simply be a hydrogen. In another embodiment, X is a sequence of up to 6 D- or L- amino acids or analogues thereof. The type of amino acids(s) should of course not affect e.g. by changing the configuration of the peptide or by changing the spatial orientation of the terminal carboxylic acid with respect to the core peptide the recognition by P-selectin in such a way that, the aimed effect is no longer obtained. Suitable analogues include amino substituted carboxylic acids, e.g. aminocyclohexanoic acid amino benzoic acid and amino caproic acid. '

Y marks the C-terminal group. Y may be a hydroxyl group. In a preferred embodiment, Y is a residue comprising 1 to 11D- or L- amino acids carrying a terminal hydroxyl group. The recognition by P-selectin is more tolerant to substituents at the C-terminus. It is also preferred that at least one amino acid with a negatively charged side chain is present in Y, preferably being separated from the remaining X(Aₓ)ₘA₃A₁A₂A₁ sequence by one to three amino acids. In a particularly preferred embodiment of the invention Y is D- or L-lysine (K), or comprises D- or L- lysine (K).

The compound of the invention can possess a cyclic or otherwise constrained backbone. In that case, X+Y together may suitably be linked through an S-S-binding, although, of course, also other types of (chemical) linkages can be present, such as an amide binding, a thioether binding, a carbamate binding or an ester binding.

The length of the group X+Y is not particularly critical as long as the core sequence is recognised by P-selectin. Generally, the minimum length of X+Y corresponds with the length of 5-6 amino acids.

The group R¹-(Z)ₙ- with which X or Y, or both X and Y independently, are substituted, may be selected from R-CO groups wherein R is an aryl or heteroalkyl group. The heteroalkyl group preferably has 2 to 8 carbon atoms, at least one of which is replaced by a nitrogen atom. An aryl group has preferably 6 to 14 carbon atoms, and the group may be substituted with at least one group selected from OH or COOH.

Examples of compounds are those wherein Z is -CO-, and R¹ the following groups: 4-hydroxyphenylcarbonyl, 3,5-dihydroxyphenylcarbonyl, 3-hydroxy-5-carbonyloxyphenylcarbonyl, 3,4,5-tri-methoxyphenylcarbonyl, 4-carboxyphenylcarbonyl, 3-carbonylbutyric acid, 3-carboxyphenylcarbonyl, However, especially preferred groups are acyl groups and more particularly 3,5-dicarboxyphenylcarbonyl and 3,4,5-trihydroxyphenylcarbonyl groups.

Further, also modifications based on tannic acid, caffeinic acid, oligohydroxyl aryl groups and oligocarboxy-aryl groups as well as derivatives thereof are preferred, especially on group X. The 3,4,5-trihydroxyphenylcarbonyl group however can be used advantageously both on group X and group Y.

It is assumed that the R¹-(Z)ₙ modification confers additional interaction of the peptide compound with the P-selectin binding pocket on P-selection, possibly by electrostatic interaction or H-bridge formation. This leads to gain inaffinity for P-selectin.

Very good results are obtained for those compounds having an N-terminal amide function. Also very good results are obtained when using a linker between the N-terminal substituent group and amino acid Aₓ or A₃ in the amino acid sequence of the compounds of the invention. Suitable linkers or spacers are glycine and aminobutyric acid.

Compounds of the invention possess linear, branched, cyclic, or constrained backbones. For instance, peptides with at least two cysteine (C) units can be cyclisised by oxidation. If a compound is cyclisised via such a disulfide bond, it is preferred that the participating cysteine units are members of X and Y, respectively. Cyclic structures are, in fact, an example for conformationally constrained backbones. Other types of constrained structures may also be introduced to decrease the conformational flexibility of the compound. Especially the presence of olefinic bonds or small ring structures in the backbone serves this purpose. Examples of such constraints are given in Pharmaceutical Biotechnology, Ed. D.J.A. Crommelin and R.D. Sindelar, Harwood Academic Publishers, 1997, p. 139f.

In one of the preferred embodiments, the compounds of the invention are provided as multimers of the derivatised peptides.

In another type of multimer that can be created to form the compounds of the invention, single derivatised peptide chains in accordance with the invention are coupled to a biocomapatible protein, such as human serum albumin, humanised antibodies, liposomes, micelles, synthetic polymers, nanoparticles, and phages. Multimers can also represent derivatised peptides which are serially coupled to each other via spacers, i.e. concatamers, or dendrimers, or clusters.

The compounds can generally be prepared by the methods that are known for the preparation of peptides and derivatives thereof. Smaller compounds containing only a few amino acids and preferably not more than 30-50 units, can be prepared by chemical and/or enzymatic ligation techniques, either using the classical approach in which the reactions take place in solution or suspension, or by employing the more modern solid phase approach, in which the peptide is assembled while being anchored to a solid surface, such as a polymeric bead. Larger compounds are typically synthesised by automatic solid phase peptide synthesisers. The compounds obtained are then modified by reaction with a suitable R¹(Z)ₙ- source, preferably a R¹(Z)ₙ- OH group. Said source reacts with a primary amine group in the peptide compound.

When the peptide sequence in the compounds according to the invention is made by standard Fmoc chemistry and the protecting Fmoc group is removed, the N-terminus is immediately available for the reaction introducing the R¹(Z)ₙ-group.

When the C-terminus has no free amine available, the said source can be reacted with an added lysine, which lysine can suitably be added as its DDE derivative. DDE is 4,4-dimethyl-2,6-dioxocyclohex-1-ylidene)-ethyl; it can selectively be removed with 2% hydrazine without affecting acid-labile side chain protecting groups.

Some more comprehensive summaries of methods which can be applied in the preparation of the compounds are described in: W.F. Anderson, Nature 392 Supp., 30 April 1998, p. 25-30; Pharmaceutical Biotechnology, Ed. D.J.A. Crommelin and R.D. Sindelar, Harwood Academic Publishers, 1997, p. 53-70,167-180, 123-152, 8-20; Protein Synthesis; Methods and Protocols, Ed. R. Martin, Humana Press, 1998, p. 1-442; Solid-Phase Peptide Synthesis, Ed. G.B. Fields, Academic Press, 1997, p. 1-780; Amino Acid and Peptide Synthesis, Oxford University Press, 1997, p. 1-89.

Salts of the derivatives of the peptides are prepared by known methods, which typically involve the mixing of the derivative of the peptide with either a pharmaceutically acceptable acid to form an acid addition salt, or with a pharmaceutically acceptable base to form a base addition salt. Depending on the intended use pharmaceutically acceptable acids include organic and inorganic acids, such as formic acid, acetic acid, propionic acid, lactic acid, glycolic acid, oxalic acid, pyruvic acid, succinic acid, maleic acid, malonic acid, cinnamic acid, sulfuric acid, hydrochloric acid, hydrobromic acid, nitric acid, perchloric acid, phosphoric acid and thiocyanic acid, which form ammonium salts with free amino groups of the derivatised peptides. Pharmaceutically acceptable bases, which form carboxylate salts with free carboxylic groups of the derivatised peptides include ethylamine, methylamine, dimethylamine, triethylamine, isopropylamine, diisopropylamine, and other mono-, di- and trialkylamines, as well as arylamines.

Moreover, also pharmaceutically acceptable solvates are encompassed.

Multimers of the derivatised peptides according to the invention can, for example, be prepared in a similar way as multimers of peptides, e.g. by biotinylating the N-terminus chains and subsequent complexation with streptavidin, thereby if deemed necessary adjusting the method to the specific compounds. As streptavidin is able to bind 4 biotin molecules or conjugates with high affinity, very stable tetrameric peptide complexes can be formed by this method. Multimers may be composed of identical or different derivatised peptides. Preferably, however, the multimers of the invention are composed of two or more identical derivatised peptides.

The compounds of the invention have an affinity to human P-selectin, a membrane glycoprotein expressed by vascular endothelial cell and platelets, which is involved in leukocyte adhesion to the endothelium and platelets. The affinity or binding characteristics of compounds to P-selectin can be quantified, for example, in terms of the IC₅₀-value. Typically, an IC₅₀-value of about 50-100 µM or less would be considered as evidence for affinity and binding. More desirable for ligands are substances with IC₅₀-values of about 10 µM or less. The lowest a IC₅₀-value attainable for the non-covalent type bonds playing a role in the interactions or bindings in accordance with the present invention is about 10⁻¹⁵ M. Generally, however, the IC₅₀-values are higher than about 10⁻¹² M and in most cases higher than about 10⁻⁹ M.

The compounds of the invention have low micromolar to low nanomolar IC₅₀-values towards P-selectin, which make them about equally potent as the natural ligand P-selectin glycoprotein ligand-1.

It was found that N-terminally modified compounds of the invention were more effective (potent and/or specific) than the corresponding C-terminally modified compounds containing the same modifications. Very good results were obtained with modification by 1,3,5-tricarboxylic acid or gallic acid.

In addition thereto the compounds according to the invention may render the peptides more stable against proteases, particularly against N-exopeptidases, due to the derivatisation. The compounds further may have an enhanced specificity for P-selectin and therefore modulate a pharmacological action such as interaction between monocyte and platelet, that between monocyte and endothelial cells, that between platelet cells and platelet cells. Potential side effects may thus also be diminished. The compounds would be cross-reactive in many species.

A further aspect of the invention refers to the uses of the disclosed compounds. Since the compounds bind selectively to P-selectin, they can, depending on their type of interaction with P-selectin after binding, function as antagonists, partial antagonists, or as mere targeting means to target conjugated substances to cells and tissues expressing P-selectin. Thus, the compounds can be advantageously used in pharmaceutical compositions.

Pharmaceutical compositions containing the compounds of the invention may be adapted for various routes of administration, such as parenteral, oral, transmucosal, nasal, or pulmonary. They may further contain drug targeting agents, bioavailability enhancement agents, or active ingredients other than compounds of the invention, and provide for immediate or modified release.

As used herein, the term "pharmaceutical composition" refers to therapeutic and diagnostic compositions, as well as to medicaments and diagnostics containing such compositions. Therapeutic compositions and medicaments are used for the prevention or treatment of diseases and other conditions of mammals of which conditions improvement is desirable. Diagnostics and diagnostic compositions are used for the diagnosis of such diseases *in vivo* and *in vitro.*

A preferred use of the compounds is for preparing therapeutic compositions or medicaments to prevent or improve diseases and conditions involving the adhesion of leukocytes, such as monocytes and neutrophils, to the vascular endothelium and to platelets. The compounds can also be used in compositions for treating diseases in which the inhibition of P-selectin-mediated intracellular signaling is desirable.

For instance, compositions containing one or more compounds of the invention can contribute to controlling leukocyte-mediated inflammatory processes. It is known that activated leukocytes release toxic molecules which can damage normal tissue. These inflammatory responses, some of which also involve P-selectin-mediated platelet activation, are part of several pathological conditions, such as transplant rejection, cold ischemia, hemorrhagic shock, septic shock, tumour metastasis, thrombosis, chronic inflammation, rheumatoid arthritis, inflammatory bowel disease, atherosclerosis, restenosis, angiogenesis, disseminated intravascular coagulation, adult respiratory distress syndrome, circulatory shock, severe traumatic brain injury, relapsing-remitting multiple sclerosis, cerebral artery occlusion, ischemia, stroke, acute myocardial infarct, deep vein thrombosis, arterial injury, such as produced by angioplasty, myocardial ischemia, renal injury from ischemia and reperfusion, and renal failure.

In another embodiment, the compounds are used in the preparation of diagnostic compositions or products. Such compositions can be used for *in vitro* tests to quantify P-selectin concentrations in body fluids as markers for the diseases and conditions described above. They may be also used for *in vivo* diagnostic imaging procedures to monitor P-selectin mediated atherosclerosis, aneurisms, restenosis following percutaneous transluminal coronary angioplasty (post-PTCA restenosis) and other conditions selected from those in which P-selectin is mobilised. As an option for this use a compound according to the invention may be conjugated with a chelator, which is subsequently complexed with an isotropic label that is detectable by the chosen monitoring system.

Another use of the compounds is as that of a tool in research. For instance, they can be used to test the binding affinity of molecules to P-selectin. To conduct this test method, P-selectin would be contacted and incubated with a molecule to be tested for binding affinity and with a compound of the invention. A reduced binding of the compound of the invention would indicate an affinity of the molecule to P-selectin.

The compounds can also be used as targeting molecules or conjugates in pharmaceutical compositions for the targeting of drugs or genetic material to tissues that express P-selectin. As conjugates, the compounds can be directly coupled with active molecules or nucleic acids that are to be delivered to such tissues. Alternatively, they can be incorporated into or anchored onto the surface of liposomes or other lipid vesicles, emulsion droplets, polymers, nano- or microparticles to obtain targeted vehicles for drugs or genetic material which is delivered to P-selectin expressing tissues.

The pharmaceutical compositions preferably contain one or more compounds with P-selectin affinity as disclosed herein and at least one carrier or excipient. As used herein, a carrier or excipient is any pharmaceutically acceptable substance or mixture of substances having no substantial pharmacological activity, which can be used as a vehicle or as an auxiliary substance to formulate a compound into dosage form which is stable and easy to administer. Examples of pharmaceutically acceptable excipients are found in the monographs of all major pharmacopoeias.

In one embodiment, the composition is formulated and processed for parenteral injection, preferably for intravascular injection, such as intravenous or intra-arterial, but also for intramuscular, subcutaneous, intralesional, intraperitoneal or other routes of parenteral administration. The same principles that govern the formulation of other drugs for these administration routes will also teach those skilled in the arts on how to prepare such compositions. For instance, one of the requirements of parenteral dosage forms is their sterility. Other requirements are described in all major pharmacopoeias, such as in USP 24, in the monograph "General Requirements for Tests and Assays. 1. Injections", p. 1775-1777. To increase the stability of a parenteral formulation, it may be necessary to provide a dried dosage form which must be reconstituted before it can be administered. An example of such a dosage form is a freeze-dried or lyophilised formulation.

It may be desirable to administer a compound of the invention as a parenteral controlled release dosage form to avoid frequent injections and to improve the effectiveness and convenience of the therapy. Various methods of preparing such depot formulations are known. Prolonged release may be provided by solid implants, nanoparticles, nanocapsules, microparticles, microcapsules, emulsions, suspensions, oily solutions, liposomes, or similar structures.

Excipients that are particularly useful for the preparation of parenteral formulations are solvents, cosolvents and liquid or semisolid carriers, such as sterile water, ethanol, glycerol, propylene glycol, polyethylene glycol, butanediol, fatty oils, short- and medium chain triglycerides, lecithin, polyoxyethylene castor oil derivatives; substances to adjust the osmolality and pH, such as sugars, especially glucose, sugar alcohols, especially mannitol, sodium chloride, sodium carbonate, citric acid, acetate, phosphate, phosphoric acid, hydrochloric acid, sodium hydroxide etc.; stabilizers, antioxidants, and preservatives, such as ascorbic acid, sodium sulfite or -hydrogen sulfite, EDTA, benzyl alcohol etc.; other excipients and lyophilization aids, such as albumin, dextran etc.

Alternatively, the pharmaceutical compositions may be designed for oral administration and processed accordingly. Appropriate oral dosage forms include tablets, hard capsules, soft capsules, powders, granules, orally disintegrating dosage forms, syrups, drops, suspensions, effervescent tablets, chewable tablets, oral films, lyophilised dosage forms, sustained release dosage forms and controlled release dosage forms. In one of the preferred embodiments, the oral dosage form is an enterically coated solid dosage form to provide protection of the compound from the acidic and proteolytic environment of the stomach.

It may also be advantageous to administer a compound of the invention in a transmucosal dosage form. This route of administration is non-invasive and patient-friendly; at the same time it may lead to an improved bioavailability of the compound compared to oral administration, especially if the compound is not stable in the fluids of the digestive system, or if it is too large to be absorbed from the gut effectively. Transmucosal administration is possible via, for instance, nasal, buccal, sublingual, gingival or vaginal dosage forms. These dosage forms can be prepared by known techniques; they can be formulated to represent nasal drops or sprays, inserts, films, patches, gels, ointments or tablets. Preferably, the excipients used for a transmucosal dosage form include one or more substances providing for mucoadhesion, thus prolonging the contact time of the dosage form with the site of absorption and thereby potentially increasing the extent of absorption.

In a further embodiment, the compounds are administered via the pulmonary route, using a metered dose inhaler, a nebuliser, an aerosol spray, or a dry powder inhaler. Appropriate formulations can be prepared by known methods and techniques. Transdermal, rectal, or ocular administration may also be feasible in some cases.

It can be advantageous to use advanced drug delivery or targeting methods to deliver a compound of the invention more effectively. For instance, if a non-parenteral route of administration is chosen, an appropriate dosage form may contain a bioavailability enhancing agent, which may be any substance or mixture of substances which increases the availability of the compound. This may be achieved, for instance, by the protection of the compound from degradation, such as by an enzyme inhibitor or an antioxidant. More preferably, the enhancing agent increases the bioavailability of the compound by increasing the permeability of the absorption barrier, which is typically a mucosa. Permeation enhancers can act via various mechanisms; some increase the fluidity of mucosal membranes, while others open or widen the gap junctions between mucosal cells. Still others reduce the viscosity of the mucus covering the mucosal cell layer. Among the preferred bioavailability enhancers are amphiphilic substances such as cholic acid derivatives, phospholipids, ethanol, fatty acids, oleic acid, fatty acid derivatives, EDTA, carbomers, polycarbophil, and chitosan.

In a further aspect, molecules capable of binding to the compounds disclosed above are within the scope of the invention. For instance, standard hybridysation technology can be applied to prepare specific monoclonal antibodies to a compound Other techniques are available to design and prepare smaller molecules capable of binding to a compound of the invention.

The following examples further illustrate the invention, but are not intended to limit its scope to the embodiments presented therein.

### EXAMPLES

### Example 1

### Chemical synthesis of resin-bound core sequence compound 9

Core sequence compound 9 (FmocHN-Glu(OtBu)-Trp(Boc)-Val-Asp(OtBu)-Val-Lys(DDE)-GABA-HMPA-resin; see fig. 1) was synthesised on an Applied Biosystems 9050 peptide synthesizer (Warrington, UK) using standard Fmoc chemistry. In short, Tentagel S-NH₂ (load 0.26 mmol/g) was provided with 4-hydroxymethylphenoxyacetic acid (HMPA) as a linker, resulting in compound 8 (see fig. 1). Fmoc-GABA -OH (10 eq.) was attached to the HMPA resin 8 under the agency of N,N'-dicyclohexylcarbodiimide (DCC, 5 eq.) and 4-dimethylaminopyridine (DMAP, 0.5 eq.) All other amino acids, with acid labile side chain protection if necessary, were attached by coupling in the presence of HOBt/TBTU/Dipea (4/4/8 eq.). After coupling, the resin was washed with DMF, isopropanol and diethylether and subsequently dried.

### Example 2

### Synthesis of compound 10

The solid phase synthesis of the compound library 10 (see fig. 1) was performed using a Flexchem^{®} system (Robbins Scientific, Sunnyvale U.S.A.). After removal of the N-terminal Fmoc group of compound 9 by 20% piperidine in DMF, the resin was washed (DMF) and dried. The resin was distributed in 10 mg quantities over a solvent-resistant 48-well filter plate. After washing with DMF, a mixture of the desired carboxylic acid (40 eq.), BOP (20 eq.), HOBt (20 eq.) and NMM (100 eq.) was added (total volume 300 µl) and the suspended resin was incubated for 3 hours under continuous perspiration with N₂. Subsequently, the resin was washed with DMF and incubated three times for 3 minutes with hydrazine monohydrate (2% in DMF) to remove the DDE (4,4-dimethyl-2,6-dioxocyclonhex-1-ylidene)ethyl) group. After washing with DMF, a mixture of the second carboxylic acid, BOP, HOBt and NMM (same amounts as described above) was added and once again incubated for 3 hours. Derivatised peptides, which were only modified at the C-terminal lysine (HP*01*-HP*07* see hereafter), were N-boculated with di-t.-butyl-dicarbonate ((t-Boc)₂ O; 0.25 M) and Dipea (0.125 M in NMP) prior to reaction to protect the N-terminal amine function. After removal of the solvent, peptides were cleaved off from the resin with a mixture of trifluoro acetic acid (TFA), triisopropylsilane (TIS) and water (95:2.5:2.5 v/v/v). Each sample was lyophilised and stored at -20°C until use.

### Example 3

### Synthesis of compounds 11-13, Compound Library 14 and compounds 27-33

Derivatised peptides 11-13, compound library 14 and derivatised peptides 27-33 were prepared in the same manner as described for library 10 from resin bound core sequence FmocHN-Trp(Boc)-Val-Asp(OtBu)-Val-HMPA-resin. Derivatised peptide 11 was acetylated using acetic anhydride (0.25 M) and Dipea (0.125 M) in NMP.

### Example 4:

### Competition assay with TM11-PO

Compounds according to the invention, as prepared in examples 1, 2 and 3 and some reference peptide compounds were assayed for its ability to inhibit TM11-PO binding to human P-selectin TM11-PO, a tetrameric TM11/strepPO complex, which has previously been shown to bind with high affinity and specificity to human P-selectin (Molenaar et al., Blood 2002, 100, 3570-3577), was freshly prepared by incubating streptavidin-peroxidase (strep-PO (Amersham Life Science, Little Chalfont, United Kingdom), 8.4 µl, 2.0 µM) and TM11-biotin (biotin-CDVEWVDVSSLEWDLPC (synthesised by Dr. Van der Zee, Department of Immunology, University of Utrecht, Utrecht, the Netherlands), 1.5 µl 190 mM) for 2 hours at room temperature in assay buffer (20 mM HEPES, 150 mM NaCl, 1 mM CaCl₂, pH 7.4). For competition studies, a 96 wells microtiter plate (high binding, flat bottom, Costar, Coming, U.S.A.) was coated overnight at 4°C with 10 µg/ ml goat antihuman IgG (Fc specific) (Sigma-Aldrich, Zwijndrecht, the Netherlands) in coating buffer (50 mM NaHCO₃, pH 9.6). Subsequently, wells were washed with assay buffer and incubated for 1 hour at 37°C with blocking buffer (3% BSA in assay buffer). After washing with assay buffer, the wells were incubated for 2 hours at 37°C with the human P-selectin IgG-Fc chimera (R&D Systems Europe Ltd., Abingdon, United Kingdom) (0.3 µg/ml). Subsequently, wells were washed with assay buffer and incubated for 1 hour at 4°C with the TM11 -PO complex. The wells were washed six times with washing buffer (0.1 % Tween 20 in assay buffer). 3,3',5,5'-Tetramethylbenzamidine (TMB)/ hydrogen peroxide (H₂O₂) (Pierce, Rochford, U.S.A.) was added and wells were incubated at room temperature for 15 minutes. The reaction was halted by addition of 2 M H₂SO₄ and the absorbance was measured at 450 nm.
For the results see the tables below and figure 3.

**Table 1: Derivatised peptide and IC₅₀ (µM)**

| | |
|---|---|
| HP*14* | 1.02 |
| HP*16* | 0.31 |
| *HP17* | 0.45 |
| HP*61* | 0.19 |
| HP*62* | 0.28 |
| HP*63* | 3.40 |
| HP*65* | 2.35 |
| *HP60* | 1.10 |
| *HP06* | 6.00 |

The derivatised peptides are coded HP*ij*, wherein *i* and *j* refer to the acyl moieties R¹ and R² attached to the N- and C-terminus, respectively, of formula 10 (Fig.1). Unmodified amino group is indicated by *0*. The acyl moieties 1 to 7 are shown in Fig. 2.

The affinity of the compounds was confirmed by a cell adhesion assay in which Chinese hamster ovary cells expressing human P-selectin were incubated with HL60 cells expressing PSGL-1 in the presence of titered amounts of the compounds.

**Table 2: Derivatised peptides and their IC₅₀-values for human P-selectin as determined by competition ELISA**

| **Peptide** | **Sequence** | **IC₅₀(µM)^{a}** |
|---|---|---|
| TM11 | CDVEWVDVSSLEWDLPC | 2^{b} |
| A29 | EWVDV | 8^{b} |
| A129 | DWVDV | 16^{b} |
| A130 | KWVDV | 28^{b} |
| A131 | AWVDV | 27^{b} |
| A138 | WVDV | > 1000^{b} |
| 11 | Ac-WVDV | 27 |
| 12 | GA-WVDV | 0.037 |
| 13 | GA-EWVDV | 0.031 |

| | | |
|---|---|---|
| ^{a} results are the average of at least 3 experiments at 8 different concentrations of peptide as determined in TM11-PO competition ELISA (materials and methods) ^{b}from not pre-published European patent application 01203314.8. | | |

**Table 3: Gallic acid derivatized peptides, and their IC₅₀ for human P-selectin as determined by competition ELISA.**

| **Peptide** | **Structure** | **IC₅₀(nM)^{a}** |
|---|---|---|
| 12 | 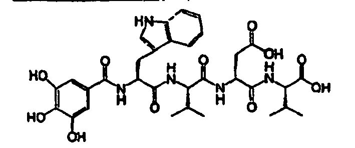 | 37.1 |
| 27 | 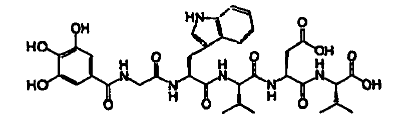 | 18.3 |
| 28 | 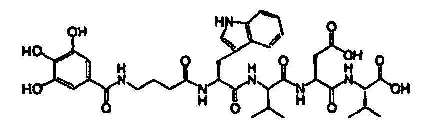 | 15.4 |
| 29 | 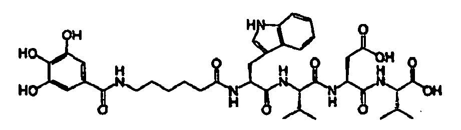 | 62.9 |
| 30 | 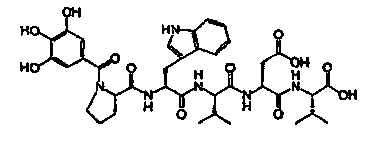 | 250 |
| 31 | 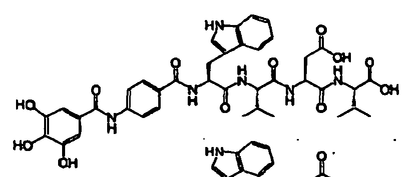 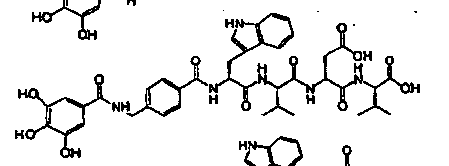 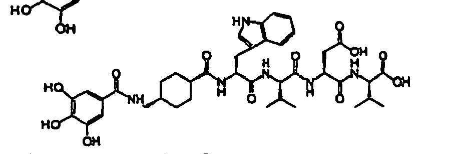 | >1000 |
| 32 | | 590 |
| 33 | | 580 |

| | | |
|---|---|---|
| ^{a} values are the mean of at least 3 experiments at 8 different concentrations of peptide as determined in TM 11 -PO competition ELISA (materials and methods) | | |

### Example 5

### Competition assay with PAA-Le^{a}-SO₃H

To be able to test the binding of compounds according to the invention also to other members of the selectin family (i.e. E- and L-selectin), biotin-PAA-le^{a}-SO₃H (Synthesone, Munich, Germany) instead of TM11-PO was used as ligand in the competition assay. This polyacrylamide (PAA) based polymer with attached sulfo-Lewis A groups, is an established selectin ligand.

Experimentally, the same procedure was used as in example 1 however with the following differences. After washing with assay buffer, the wells were incubated for 2 hours at 37°C with the human P-selectin IgG-Fc chimera, mouse P-selectin IgG-Fc chimera, human L-selectin IgG-Fc chimera and human E-selectin IgG-Fc chimera respectively (all from R&D Systems Europe Ltd., Abingdon, United Kingdom) (0.3 µg/ml). Subsequently the wells of the microtiter plate were incubated with biotin-PAA-Le^{a}-SO₃H (0.33 µg/ml) for 2 hours at 37°C, instead of with the TM11-PO-complex.

For the results see figure 7.

### Example 6

### Inhibition of dynamic interactions between HL60 cells and human P-selectin expressing Chinese hamster ovary cells

Chinese hamster ovary (CHO) cells stably transfected with human P-selectin (CHO-P) were kindly donated by Dr. Modderman (University of Amsterdam, Amsterdam, the Netherlands). Cells were grown in DMEM (BioWhittaker Europe, Verviers, Belgium) containing 10% foetal calf serum (FCS) (BioWitthaker), 5 mM L-glutamine, 20,000 units penicillin/streptomycin (Bio Whittaker) and 5 mM non-essential amino acids (Gibco, Paisley, United Kingdom). Flasks with cells were incubated at 37°C in 5% CO₂ for 3 or 4 days until cells had grown nearly confluent.

HL60 cells were from ATCC and grown in RPMI 1640 medium (Bio Whittaker) with 10% FCS, 5mM L-glutamine and 20,000 units penicillin/streptomycin.

HL-60 cells were fluorescently labeled by incubation for 30 min at 37°C with 5 µM calcein-AM (Molecular Probes, Leiden, The Netherlands) in RPMI. These cells (50,000/well) were added to cultured CHO cells expressing P-selectin (CHO-P cells, cultured in DMEM with 10% FCS, 5 mM non-essential amino acids, 5 mM L-glutamine and 20,000 U penicillin/streptomycin), seeded in 96 well plates in the presence or absence of the P-selectin antagonists (1h, 4°C). After gentle washing with RPMI, CHO-P associated fluorescence was measured (λ_{exc} 485/ λ_{cm} 530 nm).

For the results see figure 8.

## Claims

1. A compound with affinity to human P-selectin, which is a derivative of a peptide represented by sequence X(Aₓ)ₘA₃A₁A₂A₁Y, wherein:
- A₁ is D- or L-cysteine (C), or D- or L-valine (V);
- A₂ is D- or L-aspartic acid (D);
- A₃ is D- or L-phenylalanine (F), or a D- or L-tryptophan (W);
- Aₓ is a D- or L-amino acid, selected from the group consisting of glutamic acid (E), aspartic acid (D), glycine (G) and cysteine (C);
- X marks the N-terminal side of said sequence and is hydrogen or a residue comprising 1 to 6 D- or L-amino acids or analogues thereof;
- Y marks the C-terminal side of said sequence and is -OH or a residue comprising 1 to 11 D- or L-amino acids or analogues thereof;
wherein X and Y together may form a cyclic system;
**characterised in that** at least one of X and Y or X+Y is substituted with the group R¹(Z)ₙ-
wherein:
- Z is -CO- and
wherein R¹ is selected from:
a) a (C₂-C₈)alkyl group, wherein at least one C-atom is replaced with a nitrogen atom;
b) a (C₆-C₁₄)aryl group, which may be substituted with at least one group selected from -OH or -COOH;
and wherein m and n are integers independently selected from 0 and 1.

2. The compound according to claim 1, wherein Aₓ represents D- or L-glutamic acid (E) or D- or L- aspartic acid.

3. The compound according to claim 1 or 2, wherein A₁ represents D- or L-valine (V).

4. The compound according to any one of the preceding claims, wherein A₃ is D- or L-tryptophan (W).

5. The compound according to any one of the preceding claims, wherein Y is a residue comprising D- or L- lysine.

6. The compound according to any one of the preceding claims, wherein R¹ is unsubstituted phenyl or phenyl substituted with at least one substituent as defined in claim 1.

7. The compound according to any one of the preceding claims, wherein n is 0 and R¹ is 3,4,5-trihydroxyphenylcarbonyl or 3,5-dicarboxyphenylcarbonyl.

8. The compound according to any one of the preceding claims, wherein X comprises no amino acids and Y comprises D- or L-lysine.

9. The compounds according to claim 8, wherein n is 0 and R¹ is 3,4,5-trihydroxyphenylcarbonyl or 3,5-dicarboxyphenylcarbonyl.

10. The compound of claim 1, wherein m is 0, wherein Z is -CO-, and wherein Z is attached to Y via a D- or L-glycine or aminobutyric acid spacer.

11. The compound according to any one of the preceding claims, comprising a cyclic or constrained backbone structure.

12. A composition comprising one or more compounds according to any one of the preceding claims.

13. A method for the preparation of a compound according to any one of any one of the preceding claims, comprising a sequence of steps wherein amino acid monomers, amino acid oligomers, or mono- or oligomers of amino acid analogues or mimetics are assembled by chemical or enzymatic ligation, and wherein said steps are performed in a liquid phase and/or at the interface to a functionaliSed solid phase.

14. The method according to claim 13, comprising reacting the HMPA linker of the formula 8 (Fig. 1) by standard Fmoc chemistry to yield a compound of the sequence X(Aₓ)ₘ A₃A₁A₂A₁Y. wherein X, Aₓ, A₃, A₁, A₂,Y and m are as defined in claim 1, and wherein the amino groups are initially protected by protecting groups, and R-CO is introduced by replacing the protecting groups by using standard methods.

15. Compound according to any one of claims 1 to 11 for use in therapy or diagnosis.

16. Use of a compound according to any one of claims 1 to 11 for the manufacture of a medicament for inhibiting leukocyte binding to platelets and/or endothelial cells.

17. The use according to claim 15 or 16 for the manufacture of a medicament for the treatment, prevention, or diagnosis of chronic inflammatory disorders, rheumatoid arthritis, inflammatory bowel disease, multiple sclerosis, atherosclerosis, restenosis, ischemia, reperfusion injury including renal failure, tumour metastasis, bacterial sepsis, disseminated intravascular coagulation, adult respiratory distress syndrome, stroke, angiogenesis, transplant rejection, thrombosis, or circulatory shock.

18. Pharmaceutical composition, comprising a compound according to any one of claims 1 to 11 and one or more pharmaceutically acceptable carriers or excipients.

19. Pharmaceutical composition according to claim 18, which is formulated and processed for parenteral administration, preferably for intravascular, intramuscular, subcutaneous or intralesional injection.

20. Pharmaceutical composition according to claim 18, which is formulated and processed for oral administration, preferably in form of a tablet, a capsule, granules, an enteric solid dosage form, a solid dosage form providing sustained or controlled release or an orally disintegrating dosage form.

21. Pharmaceutical composition according to claim 18, which is formulated and processed for transmucosal administration, such as nasal, buccal, sublingual or vaginal administration.

22. Pharmaceutical composition according to claim 18, which is formulated and processed for pulmonary administration through a metered dose inhaler, a nebulizer, an aerosol spray dispenser or a dry powder inhaler.

23. Pharmaceutical composition according to any one of claims 18 to 22, further comprising a drug targeting agent and/or a bioavailability enhancing agent.

24. A method for determining whether a molecule comprises a binding affinity for P-selectin comprising contacting P-selectin with said molecule and with a compound according to any one of claims 1-11 and determining whether binding of said compound to said P-selectin is reduced.

25. A binding molecule capable of specifically binding a compound according to any one of claims 1-11, comprising an antibody or a functional part thereof

## Patentansprüche

1. Verbindung mit Affinität zu humanem P-Selectin, welche ein Derivat eines Peptids, wiedergegeben durch die Sequenz X(Aₓ)ₘA₃A₁A₂A₁Y, ist, worin:
- A₁ D- oder L-Cystein (C) oder D- oder L-Valin (V) ist;
- A₂ D- oder L-Asparaginsäure (D) ist;
- A₃ D- oder L-Phenylalanin (F) oder D- oder L-Tryptophan (W) ist;
- Aₓ eine D- oder L-Aminosäure ist, ausgewählt aus der Gruppe, bestehend aus Glutaminsäure (E), Asparaginsäure (D), Glycin (G) und Cystein (C);
- X die N-terminale Seite der Sequenz markiert und Wasserstoff oder ein Rest, umfassend 1 bis 6 D- oder L-Aminosäuren oder Analoga davon, ist;
- Y die C-terminale Seite der Sequenz markiert und -OH oder ein Rest, umfassend 1 bis 11 D- oder L-Aminosäuren oder Analoga davon, ist;
worin X und Y zusammen ein cyclisches System bilden können;
**dadurch gekennzeichnet , dass** wenigstens einer von X und Y oder X + Y mit der Gruppe
R¹-(Z)ₙ-
substituiert ist, worin:
- Z -CO- ist und
worin R¹ ausgewählt ist aus:
a) einer (C₂-C₈) -Alkylgruppe, worin wenigstens ein C-Atom durch ein Stickstoffatom ersetzt ist;
b) einer (C₆-C₁₄)-Arylgruppe, die mit wenigstens einer Gruppe, ausgewählt aus -OH oder -COOH, substituiert sein kann;
und worin m und n ganze Zahlen sind, die unabhängig ausgewählt sind aus 0 und 1.

2. Verbindung nach Anspruch 1, worin Aₓ D- oder L-Glutaminsäure (E) oder D- oder L-Asparaginsäure darstellt.

3. Verbindung nach Anspruch 1 oder 2, worin A₁ D- oder L-Valin (V) darstellt.

4. Verbindung nach einem der vorstehenden Ansprüche, worin A₃ D- oder L-Tryptophan (W) ist.

5. Verbindung nach einem der vorstehenden Ansprüche, worin Y ein Rest, umfassend D- oder L-Lysin, ist.

6. Verbindung nach einem der vorstehenden Ansprüche, worin R¹ unsubstituiertes Phenyl oder Phenyl, substituiert mit wenigstens einem Substituenten, wie in Anspruch 1 definiert, ist.

7. Verbindung nach einem der vorstehenden Ansprüche, worin n 0 ist und R¹ 3,4,5-Trihydroxyphenylcarbonyl oder 3,5-Dicarboxyphenylcarbonyl ist.

8. Verbindung nach einem der vorstehenden Ansprüche, worin X keine Aminosäuren umfasst und Y D- oder L-Lysin umfasst.

9. Verbindung nach Anspruch 8, worin n 0 ist und R¹ 3,4,5-Trihydroxyphenylcarbonyl oder 3,5-Dicarboxyphenylcarbonyl ist.

10. Verbindung nach Anspruch 1, worin m 0 ist, worin Z -COist und worin Z über einen D- oder L-Glycin- oder Aminobuttersäurespacer an Y gebunden ist.

11. Verbindung nach einem der vorstehenden Ansprüche, umfassend eine cyclische oder gespannte ("constrained") Grundgerüststruktur.

12. Zusammensetzung, umfassend eine oder mehrere Verbindungen nach einem der vorstehenden Ansprüche.

13. Verfahren zur Herstellung einer Verbindung nach einem der vorstehenden Ansprüche, umfassend eine Reihe von Stufen, worin Aminosäuremonomere, Aminosäureoligomere oder Mono- oder Oligomere von Aminosäureanaloga oder -minetika durch chemische oder enzymatische Ligation zusammengesetzt werden und wobei die Stufen in einer flüssigen Phase und/oder an der Grenzfläche zu einer funktionalisierten festen Phase durchgeführt werden.

14. Verfahren nach Anspruch 13, umfassend die Umsetzung des HMPA-Linkers der Formel 8 (Fig. 1) mittels Standard-Fmoc-Chemie, um eine Verbindung der Sequenz X(Aₓ)ₘA₃A₁A₂A₁Y zu erhalten, worin X, Aₓ, A₃, A₁, A₂, Y und m wie in Anspruch 1 definiert sind und worin die Aminogruppen anfänglich durch Schutzgruppen geschützt sind und R-CO durch Ersetzen der Schutzgruppen durch Verwendung von Standardverfahren eingeführt wird.

15. Verbindung nach einem der Ansprüche 1 bis 11 zur Verwendung in der Therapie oder Diagnose.

16. Verwendung einer Verbindung nach einem der Ansprüche 1 bis 11 zur Herstellung eines Medikaments zur Inhibierung der Leukozytenbindung an Blutplättchen und/oder Endothelzellen.

17. Verwendung nach Anspruch 15 oder 16 zur Herstellung eines Medikaments zur Behandlung, Verhinderung oder Diagnose von chronischen entzündlichen Erkrankungen, rheumatoider Arthritis, entzündlicher Darmerkrankung, multipler Sklerose, Atherosklerose, Restenose, Ischämie, Reperfusionsschädigung einschließlich Nierenversagen, Tumormetastase, bakterieller Sepsis, disseminierter intravaskulärer Koagulation, Atemnotsyndrom bei Erwachsenen,Schlaganfall, Angiogenese, Transplantatabstoßung, Thrombose oder Kreislaufschock.

18. Pharmazeutische Zusammensetzung, umfassend eine Verbindung nach einem der Ansprüche 1 bis 11 und ein(en) oder mehrere pharmazeutisch verträgliche Träger oder Exzipientien.

19. Pharmazeutische Zusammensetzung nach Anspruch 18, die für parenterale Verabreichung, vorzugsweise für intravaskuläre, intramuskuläre, subkutane oder intraläsionale Injektion formuliert und bearbeitet ist.

20. Pharmazeutische Zusammensetzung nach Anspruch 18, die für orale Verabreichung, vorzugsweise in Form einer Tablette, einer Kapsel, von Körnchen, einer magensaftresistenten festen Dosierungsform, einer festen Dosierungsform, die eine anhaltende oder kontrollierte Freisetzung bereitstellt, oder einer oral zerfallenden Dosierungsform formuliert und bearbeitet ist.

21. Pharmazeutische Zusammensetzung nach Anspruch 18, die für transmukosale Verabreichung, wie nasale, bukkale, sublinguale oder vaginale Verabreichung formuliert und bearbeitet ist.

22. Pharmazeutische Zusammensetzung nach Anspruch 18, die für pulmonale Verabreichung durch ein Dosieraerosol ("metered dose inhaler"), einen Vernebler, einen Aerosolsprühdispenser oder einen Trockenpulverinhalator formuliert und bearbeitet ist.

23. Pharmazeutische Zusammensetzung nach einem der Ansprüche 18 bis 22, ferner umfassend ein Wirkstofftargetierungsmittel und/oder ein die Bioverfügbarkeit erhöhendes Mittel.

24. Verfahren zur Bestimmung, ob ein Molekül eine Bindungsaffinität für P-Selectin umfasst, umfassend das Inkontaktbringen von P-Selectin mit dem Molekül und mit einer Verbindung nach einem der Ansprüche 1-11 und Bestimmen, ob die Bindung der Verbindung an das P-Selectin verringert wird.

25. Bindemolekül, fähig zur spezifischen Bindung einer Verbindung nach einem der Ansprüche 1-11, umfassend einen Antikörper oder einen funktionellen Teil davon.

## Revendications

1. Composé ayant une affinité vis-à-vis de la P-sélectine humaine, lequel est un dérivé d'un peptide représenté par la séquence X(Aₓ)ₘA₃A₁A₂A₁Y, dans laquelle :
- A₁ est la D- ou L-cystéine (C) ou la D- ou L-valine (V) ;
- A₂ est l'acide D- ou L-aspartique (D) ;
- A₃ est la D- ou L-phénylalanine (F) ou le D- ou L-tryptophane (W) ;
- Aₓ est un acide aminé D ou L, choisi dans le groupe constitué de l'acide glutamique (E), de l'acide aspartique (D), de la glycine (G) et de la cystéine (C) ;
- X marque le côté de l'extrémité N de ladite séquence et est un hydrogène ou un résidu comprenant 1 à 6 acides aminés D ou L ou analogues de ceux-ci ;
- Y marque le côté de l'extrémité C de ladite séquence et est -OH ou un résidu comprenant 1 à 11 acides aminés D ou L ou analogues de ceux-ci ;
dans lequel X et Y peuvent former ensemble un système cyclique ;
**caractérisé en ce qu'**au moins l'un de X et Y ou X + Y est substitué par le groupe R¹-(Z)ₙ-
dans lequel :
- Z est -CO- et
dans lequel R¹ est choisi entre :
a) un groupe alkyle en C₂-C₈, dans lequel au moins un atome de C est remplacé par un atome d'azote ;
b) un groupe aryle en C₆-C₁₄, lequel peut être substitué par au moins un groupe choisi entre -OH ou -COOH ;
et dans lequel m et n sont des nombres entiers indépendamment choisis entre 0 et 1.

2. Composé selon la revendication 1, dans lequel Aₓ représente l'acide D- ou L-glutamique (E) ou l'acide D- ou L-aspartique.

3. Composé selon la revendication 1 ou 2, dans lequel A₁ représente la D- ou L-valine (V).

4. Composé selon l'une quelconque des revendications précédentes, dans lequel A₃ est le D- ou L-tryptophane (W).

5. Composé selon l'une quelconque des revendications précédentes, dans lequel Y est un résidu comprenant de la D- ou L-lysine.

6. Composé selon l'une quelconque des revendications précédentes, dans lequel R¹ est un phényle non substitué ou un phényle substitué par au moins un substituant tel que défini dans la revendication 1.

7. Composé selon l'une quelconque des revendications précédentes, dans lequel n est 0 et R¹ est le 3,4,5-trihydroxyphénylcarbonyle ou le 3,5-dicarboxyphénylcarbonyle.

8. Composé selon l'une quelconque des revendications précédentes, dans lequel X ne comprend pas d'acides aminés et Y comprend de la D-ou L-lysine.

9. Composé selon la revendication 8, dans lequel n est 0 et R¹ est le 3,4,5-trihydroxyphénylcarbonyle ou le 3,5-dicarboxyphénylcarbonyle.

10. Composé de la revendication 1, dans lequel m est 0, dans lequel Z est -CO- et dans lequel Z est attaché à Y via un groupe d'intercalation de type D- ou L-glycine ou acide aminobutyrique.

11. Composé selon l'une quelconque des revendications précédentes, comprenant une structure cyclique ou à squelette contraint.

12. Composition comprenant un ou plusieurs composés selon l'une quelconque des revendications précédentes.

13. Procédé pour la préparation d'un composé selon l'une quelconque des revendications précédentes, comprenant une séquence d'étapes dans lesquelles des monomères acides aminés, des oligomères d'acides aminés ou des monomères ou oligomères d'analogues ou mimétiques d'acides aminés sont assemblés par ligature chimique ou enzymatique et dans lequel lesdites étapes sont effectuées en phase liquide et/ou à l'interface avec une phase solide fonctionnalisée.

14. Procédé selon la revendication 13, comprenant de faire réagir le groupe de liaison HMPA de formule 8 (Fig. 1) par la chimie au Fmoc standard pour produire un composé ayant la séquence X(A_{X})ₘA₃A₁A₂A₁Y, dans laquelle X, Aₓ, A₃, A₁, A₂, Y et m sont tels que définis dans la revendication 1, et dans lequel les groupes amino sont initialement protégés par des groupes protecteurs,
et R-CO est introduit en remplaçant les groupes protecteurs en utilisant des procédés standards.

15. Composé selon l'une quelconque des revendications 1 à 11 destiné à être utilisé en thérapie ou en diagnostic.

16. Utilisation d'un composé selon l'une quelconque des revendications 1 à 11 pour la fabrication d'un médicament servant à inhiber la liaison de leucocytes aux plaquettes et/ou aux cellules endothéliales.

17. Utilisation selon la revendication 15 ou 16 pour la fabrication d'un médicament pour le traitement, la prévention ou le diagnostic de troubles inflammatoires chroniques, de la polyarthrite rhumatoïde, d'une affection abdominale inflammatoire, de la sclérose en plaques, de l'athérosclérose, de la resténose, d'une lésion d'ischémie-reperfusion dont une insuffisance rénale, d'une métastase tumorale, d'une sepsie bactérienne, d'une coagulation intravasculaire disséminée, du syndrome de détresse respiratoire de l'adulte, d'un accident vasculaire cérébral, d'une angiogenèse, d'un rejet de greffe, d'une thrombose ou d'un choc circulatoire.

18. Composition pharmaceutique, comprenant un composé selon l'une quelconque des revendications 1 à 11 et un ou plusieurs véhicules ou excipients acceptables du point de vue pharmaceutique.

19. Composition pharmaceutique selon la revendication 18, laquelle est formulée et préparée pour une administration parentérale, de préférence pour une injection intravasculaire, intramusculaire, sous-cutanée ou intralésionnelle.

20. Composition pharmaceutique selon la revendication 18, laquelle est formulée et préparée pour une administration orale, de préférence sous la forme d'un comprimé, d'une gélule, de granules, d'une forme de dosage solide gastrorésistante, d'une forme de dosage solide fournissant une libération prolongée ou contrôlée ou d'une forme de dosage se délitant oralement.

21. Composition pharmaceutique selon la revendication 18, laquelle est formulée et préparée pour une administration transmuqueuse, telle qu'une administration nasale, buccale, sublinguale ou vaginale.

22. Composition pharmaceutique selon la revendication 18, laquelle est formulée et préparée pour une administration pulmonaire via un aérosol-doseur, un nébuliseur, un pulvérisateur d'aérosol ou un inhalateur de poudre sèche.

23. Composition pharmaceutique selon l'une quelconque des revendications 18 à 22, comprenant en plus un agent de ciblage de médicament et/ou un agent augmentant la biodisponibilité.

24. Procédé servant à déterminer si une molécule présente une affinité de liaison vis-à-vis de la P-sélectine comprenant de mettre en contact la P-sélectine avec ladite molécule et avec un composé selon l'une quelconque des revendications 1-11 et de déterminer si la liaison dudit composé à ladite P-sélectine est réduite.

25. Molécule liante capable de lier spécifiquement un composé selon l'une quelconque des revendications 1-11, comprenant un anticorps ou une partie fonctionnelle de celui-ci.
